# EUROPEAN PATENT APPLICATION

(11) **EP 3 320 896 A1**
(43) Date of publication of application: **16.05.2018**
(21) Application number: 17201328.6
(22) Date of filing: 13.11.2017
(51) Int. Cl.: A61K 9/48, A61K 31/192, A61P 25/06

(54) **ORAL PHARMACEUTICAL COMPOSITIONS OF DEXKETOPROFEN AND ELETRIPTAN**

(30) Priority: 14.11.2016 TR 201616365
(71) Applicant: Sanovel Ilac Sanayi ve Ticaret A.S., 34460 Istanbul (TR)
(72) Inventor: NURIOGLU, Ayda, 34460 Istanbul (TR); MURATOGLU, Yesim, 34460 Istanbul (TR); YILDIRIM, Ediz, 34460 Istanbul (TR); TÜRKYILMAZ, Ali, 34460 Istanbul (TR)
(74) Representative: Sevinç, Erkan

(57) **Abstract**

The present invention relates to a pharmaceutical composition comprising dexketoprofen or a pharmaceutically acceptable salt thereof in combination with eletriptan or a pharmaceutically acceptable salt thereof and at least one pharmaceutically acceptable excipient.

## Description

### Field of Invention

The present invention relates to a pharmaceutical composition comprising dexketoprofen or a pharmaceutically acceptable salt thereof in combination with eletriptan or a pharmaceutically acceptable salt thereof and at least one pharmaceutically acceptable excipient.

### The background of the invention

Dexketoprofen is a non-steroidal anti-inflammatory drug (NSAID) molecule which has analgesic, anti-inflammatory and antipyretic effects. Its chemical name is (2S)-2-[3-(benzoyl)phenyl]propanoic acid and its chemical structure is shown in the Formula 1.

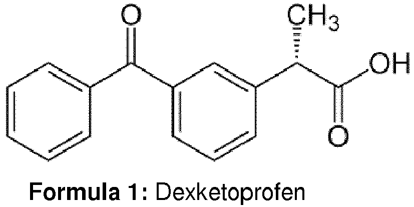

Nonsteroidal anti-inflammatory drugs (NSAIDs) are a structurally diverse group of agents with analgesic, antipyretic and anti-inflammatory properties. The primary mechanism of action of these drugs is believed to be the inhibition of enzymes of the cyclo-oxygenase (COX) family, which are involved in the biosynthesis of prostaglandins. Ketoprofen is a member of the arylpropionate group of NSAIDs, and has well established analgesic and anti-inflammatory effects. Ketoprofen is a racemic compound composed of a 50:50 mixture of its two enantiomers. As with other arylpropionic acid derivatives, it has been widely demonstrated that the inhibitory effect of ketoprofen on both isoenzymes of COX-1 and COX-2 is confined to the S(+) enantiomer (dexketoprofen), whereas the levorotatory enantiomer, or R(-)-ketoprofen, is devoid of such activity.

(+)-(S)-2-(3-Benzoylphenyl)propionic acid tromethamine salt substantially free from the (-)-(R) enantiomers of the 2-(3-benzoylphenyl)propionic acid tromethamine salts is disclosed in the patent application WO9411332A1.

5-HT is a key mediator in the pathogenesis of migraine. 5-HT1-receptor agonists, commonly known as the 'triptans', are the mainstay for acute treatment of migraine headaches.

Generally, effective triptans may be selected from a group consisting of, but not limited to sumatriptan, rizatriptan, eletriptan, naratriptan, zolmitriptan, frovatriptan, almotriptan, and functional analogs thereof, wherein the functional analogs have essentially the same biological activity.

Eletriptan (trade name Relpax®, used in the form of eletriptan hydrobromide) is a second generation triptan drug intended for treatment of migraine headaches. Its chemical name is 3-[[(2R)-1-methylpyrrolidin-2-yl]methyl]-5-(2-phenylsulfonylethyl)-1H-indole and its chemical structure is shown in the Formula 2.

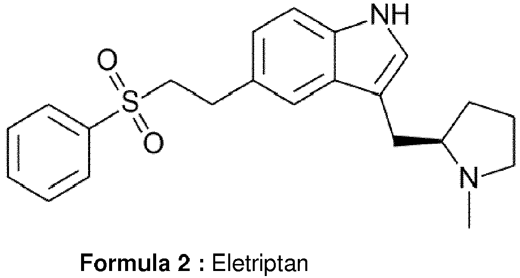

Eletriptan molecule and the use for the treatment of migraine is first disclosed in the patent application WO9206973.

Migraines are likely due to local cranial vasodilatation and/or to the release of sensory neuropeptides (vasoactive intestinal peptide, substance P and calcitonin gene-related peptide) through nerve endings in the trigeminal system. The therapeutic activity of eletriptan for the treatment of migraine headache is thought to be due to the agonist effects at the 5-HT1B/1D receptors on intracranial blood vessels (including the arterio-venous anastomoses) and sensory nerves of the trigeminal system which result in cranial vessel constriction and inhibition of pro-inflammatory neuropeptide release.

In prior art, patent application EP2306998 discloses a combination comprising a 5-HT1 agonist and a NSAID, but no patent application specifically discloses a pharmaceutical composition comprising dexketoprofen in combination with eletriptan in oral administration as tablet or capsule dosage form and there is no combination of dexketoprofen and eletriptan in the market for the treatment of migraine headache.

Thus, there is a need in the art for a pharmaceutical composition or a dosage form comprising a combination of dexketoprofen and eletriptan to achieve improved migrain headache treatment. However, some problems occur while combining these two active agents such as, physicochemical incompatibility problems.

Furthermore, in this invention, in order to overcome incompatibility and stability problems of these active agents, capsule formulations comprising mini tablet formulations for eletriptan and dexketoprofen has been developed.

### Detailed description of the invention

The main embodiment of this present invention is to provide a pharmaceutical composition for oral administration comprising dexketoprofen or a pharmaceutically acceptable salt thereof in combination with eletriptan or a pharmaceutically acceptable salt thereof and at least one pharmaceutically acceptable excipient.

According to another embodiment of the present invention, dexketoprofen or a pharmaceutically acceptable salt thereof is present in an amount of between %10 and %80 by weight of total formulation, preferably between %10 and %50, more preferably between %10 and %30, eletriptan or a pharmaceutically acceptable salt thereof is present in an amount of between %10 and %90 by weight of total formulation, preferably between %10 and %60, more preferably between %10 and %40.

According to another embodiment of the present invention, the weight ratio of dexketoprofen or acceptable salt thereof to eletriptan or acceptable salt thereof is between 0.2 and 5.0, preferably between 0.3 and 3.0, more preferably between 0.5 and 2.0.

Drugs of different action mechanisms can be combined. It is possible, however, to state that a combination of drugs having different action mechanisms, but showing actions on similar targets, will have absolutely positive effects.

While combining more than one molecule in one dosage form is increasing the patients' quality of life, improving patient adherence, many challenges also occur such as the physicochemical compatibility between the different active agents and/or between the active agents and the excipients used; and the therapeutical compatibility between the two active agents regarding their pharmacokinetic and/or pharmaceutical properties in order that the posology of the combined formulation allows to obtain safe and efficient plasma levels of both pharmacological agents.

It is well known that drugs even used in the same therapeutic area cannot always be combined in a dosage form. Using different drugs may induce incompatibility problems. The scientific literature is full of examples wherein compounds of different classes, which are used to treat the same indications, cannot be combined into safe and efficacious dosage forms thereby resulting in incompatible drug combinations. The reasons for this unexpected lack of compatibility are varied; however, it is often found that the incompatible drug combinations result in increased side effects, unwanted drug interactions or additional side effects.

In order to combine two different molecules in one dosage form, molecules should be compatible with each other to achieve desired stability and dissolution for the patients' compliance. During the development study of the present invention, it has been found that dexketoprofen and eletriptan show stability and dissolution problems due to their pH inconsistency when they used together in a tablet formulation (one layer or bilayer) or in a capsule form.

According to another embodiment of the present invention, the dosage form is in the form of a capsule, trilayer tablet, mini tablet, gelatin capsule, oral granule, sachet or a pellet, preferably a capsule.

According to this embodiment, preferably the dosage form is in the form of a capsule.

According to another embodiment, the pharmaceutical composition, in the form of a capsule comprising eletriptan mini tablets and dexketoprofen mini tablets.

According to another embodiment of the present invention, dexketoprofen mini tablets comprising 10.00 to 80.00 % by weight dexketoprofen or acceptable salt thereof as active agent, preferably between 20.00 to 60.00, more preferably between 30.00 to 40.00 and eletriptan mini tablets comprising 10.00 to 90.00 % by weight eletriptan or acceptable salt thereof as active agent, preferably between 30.00 to 70.00, more preferably 40.00 to 50.00.

According to another embodiment of this invention, dexketoprofen mini tablets or eletriptan mini tablets or both of them comprising at least one coating layer.

Suitable coating layer may also preferably be used for the protection from the moisture. It can be selected from the group comprising polyvinylpyrrolidone-vinyl acetate copolymers (Kollidon VA 64), polyvinyl alcohol-polyethylene glycol copolymers (Kollicoat IR), polyvinyl alcohol or copolymers or mixtures thereof (Opadry AMB), polymethylmetacrylate derivatives, Ethylcellulose Dispersions (Surelease), polyethylene glycol, polyvinylprolidone, polyvinylprolidone-vinyl acetate copolymer (PVP-VA) and all kinds of OpadryTM, as well as pigments, dyes, titanium dioxide, iron oxide, talc or polymethylmetacrylate copolymers (Eudragit).

According to one embodiment, coating layer is Opadry AMB which comprises polyvinyl alcohol, talc, titanium dioxide, glyceryl monocaprylocaprate, sodium lauryl sulphate, iron oxide.

In a preferred embodiment, the pharmaceutical compositon, in the form of a capsule comprises 5 dexketoprofen mini tablets and 5 eletriptan mini tablets.

The term "mini tablet", as used herein, refers to small tablets with a diameter equal to or less than 4 mm that are typically filled into a capsule or further compressed into larger tablets. Thickness of this mini tablets equal to or less than 3 mm. The mini tablets have round shape and smooth surface to ease coating process.

Mini tablets have many advantages. Some benefits of mini-tablets include excellent size uniformity, regular shape and a smooth surface. In mini tablets, smooth surface offers an excellent substrate for coating with polymeric systems. It can be concluded that pharmaceutical mini-tablets offer several advantages when compared to single unit dosage forms and are also good substitutes for granules and pellets. They have well defined size, shape, surface, low degree of porosity and high mechanical strength. Also, while combining different mini-tablets together, incompatible drugs can be administered and excipients can be selected separately for each drug.

By combining eletriptan and dexketoprofen as mini-tablets and filling them into capsules, these incompatible drugs can be administered together.

In this invention, to overcome these problems mentioned above, capsule form containing mini tablets has been developed and desired dissolution and stability has been achieved.

According to another embodiment of the present invention, the pharmaceutically acceptable excipient is selected from a group comprising disintegrants, lubricants, fillers or mixtures thereof.

Suitable lubricants are selected from a group comprising magnesium stearate, glyceryl behenate, colloidal silicon dioxide, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof, preferably the lubricants are magnesium stearate, sodium stearyl fumarate or mixture thereof.

In a preferred embodiment, the lubricant is sodium stearil fumarate in dexketoprofen mini tablet and magnesium stearate in eletriptan mini tablet.

According to this embodiment, the weight ratio of sodium stearyl fumarate in dexketoprofen mini tablet to magnesium starate in eletriptan mini tablet is between 0.1 and 10, preferably between 0.2 and 5.0, more preferably between 0.5 and 2.0.

Suitable disintegrants are selected from a group comprising starch, crospovidone (cross-linked polyvinil pyrrolidone), povidone, poloxomer, cross-linked carboxymethyl cellulose (croscarmellose sodium), low-substituted hydroxypropyl cellulose, pregelatinized starch, sodium carboxymethyl cellulose, calcium carboxymethyl cellulose, carboxymethyl cellulose, docusate sodium, guar gum, low substituted hydroxypropyl cellulose, polyacryline potassium, sodium alginate, corn starch, sodium starch glycolate, alginic acid, alginates, ion-exchange resins, magnesium aluminium silica, sodium dodesyl sulphate, poloxamer, sodium glycine carbonate, sodium lauryl sulphate or mixtures thereof, preferably the disintegrants are cross-linked carboxymethyl cellulose, corn starch, sodium starch glycolate or mixture thereof.

In a preferred embodiment, the disintegrants are corn starch and microcrystalline cellulose in dexketoprofen mini tablet and croscarmellose sodium in eletriptan mini tablet.

According to another embodiment, the weight ratio of total disintegrants in dexketoprofen mini tablet to total disintegrant in eletriptan mini tablet is between 1 and 10.

Suitable fillers are selected from a group comprising microcrystalline cellulose, mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate, polyols, dextrose, maltitol or mixtures thereof, preferably the fillers are microcrystalline cellulose, lactose monohydrate, corn starch or mixture thereof.

In a preferred embodiment, the fillers are microcrystalline cellulose and lactose monohydrate in eletriptan mini tablet and microcrystalline cellulose in dexketoprofen mini tablet.

According to another embodiment, the weight ratio of total filler in dexketoprofen mini tablet to total fillers in eletriptan mini tablet is between 0.1 and 5, preferably 0.5 and 4, more preferably 0.5 and 2.

Suitable salts of eletriptan are selected from a group comprising acetate, aspartate, benzoate, besylate, bicarbonate/carbonate, bisulphate/sulphate, hemisulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleat, malonate, mesylate, methylsulphate, naphthylate, 2-napsylate, nicotinate, nitrate, orotate, oxalate, palmitat, pamoate, phosphate/hydrogen phosphate/dihydrogen phosphate, saccharate, stearate, succinate, tartrate, tosylat and trifluoroacetate, aluminium, arginine, benzathine, calcium, choline, diethylamine, diolamine, glycine, lysine, magnesium, meglumine, lamine, potassium, sodium, tromethamine and zinc salts.

In a preferred embodiment, the pharmaceutically acceptable salt of eletriptan is eletriptan hydrobromide and the pharmaceutically acceptable salt of dexketoprofen is dexketoprofen trometamol.

In this invention, the pharmaceutical composition in the form of a capsule comprising;
a. 10.00 to 80.00 % of dexketoprofen or a pharmaceutically acceptable salt thereof
b. 10.00 to 90.00 % of microcrystalline cellulose
c. 3.00 to 50.00 % of corn starch
d. 0.4 to 20.00 % of sodium starch glycolate
e. 0.1 to 10.00 % of sodium stearyl fumarate
   by weight of the total dexketoprofen mini tablet
f. 10.00 to 90.00 % of eletriptan or a pharmaceutically acceptable salt thereof
g. 10.00 to 80.00 % of microcrystalline cellulose
h. 10.00 to 70.00 % of lactose monohydrate
i. 0.1 to 15.00 % of croscarmellose sodium
j. 0.1 to 10.00 % of magnesium stearate
   by weight of the total eletriptan mini tablet.

The process for the preparation for the oral composition of dexketoprofen and eletriptan comprises the following steps:
a. Mixing eletriptan hydrobromide, microcrystalline cellulose, lactose monohydrate, croscarmellose sodium and magnesium stearate
b. Compressing the powder mixture to form eletriptan mini tablets
c. Optionally, coating the mini tablets
d. Mixing dexketoprofen trometamol, microcrystalline cellulose, corn starch, sodium starch glycolate and sodium stearyl fumarate
e. Compressing the powder mixture to form dexketoprofen mini tablets
f. Optionally, coating the mini tablets
g. Filling the eletriptan mini tablets and the dexketoprofen mini tablets into capsules.

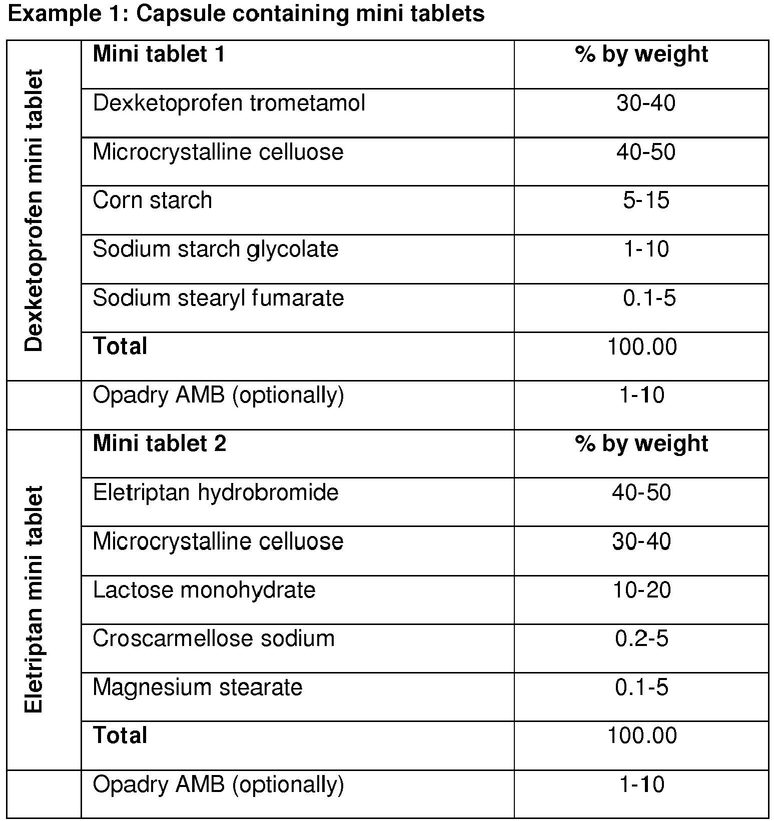

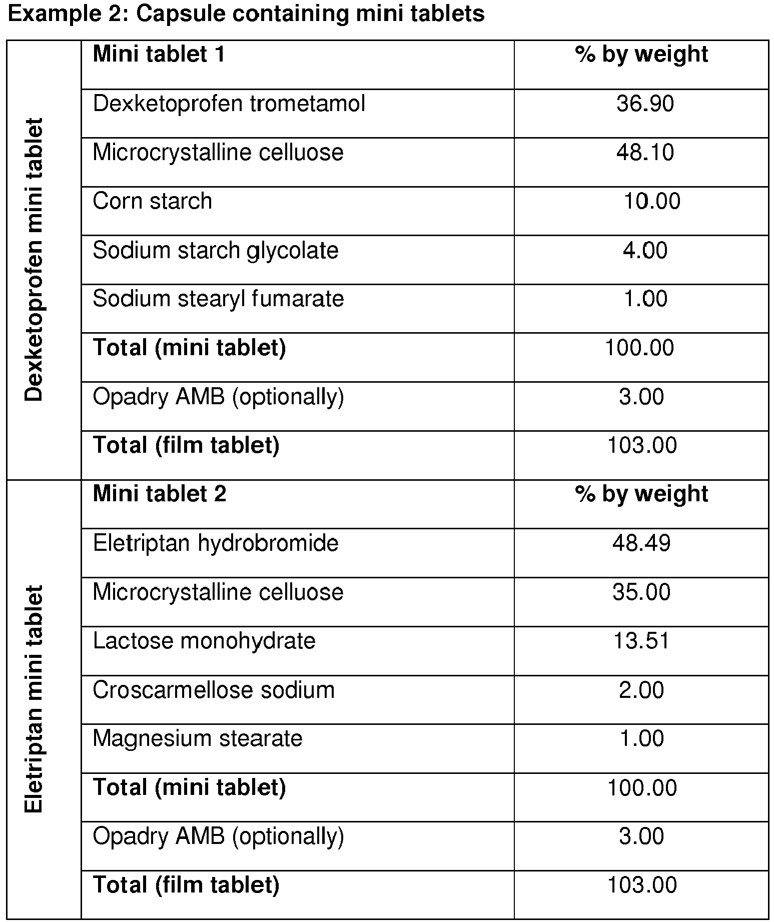

### Production process:

a. Mixing eletriptan hydrobromide, microcrystalline cellulose, lactose monohydrate, croscarmellose sodium and magnesium stearate
b. Compressing the powder mixture to form eletriptan mini tablets
c. Coating the mini tablets with Opadry AMB
d. Mixing dexketoprofen trometamol, microcrystalline cellulose, corn starch, sodium starch glycolate and sodium stearyl fumarate
e. Compressing the powder mixture to form dexketoprofen mini tablets
f. Coating the mini tablets with Opadry AMB
g. Filling the eletriptan mini tablets and the dexketoprofen mini tablets into capsules.

## Claims

1. A pharmaceutical composition comprising dexketoprofen or a pharmaceutically acceptable salt thereof in combination with eletriptan or a pharmaceutically acceptable salt thereof and at least one pharmaceutically acceptable excipient.

2. The pharmaceutical composition according to claim 1, wherein dexketoprofen or a pharmaceutically acceptable salt thereof is present in an amount of between %10 and %80 by weight of total formulation, eletriptan or a pharmaceutically acceptable salt thereof is present in an amount of between %10 and %90 by weight of total formulation.

3. The pharmaceutical composition according to claim 2, wherein the weight ratio of dexketoprofen or a pharmaceutically acceptable salt thereof to eletriptan or a pharmaceutically acceptable salt thereof is between 0.2 and 5.0, preferably between 0.3 and 3.0, more preferably between 0.5 and 2.0.

4. The pharmaceutical composition according to any of the preceding claims, wherein the dosage form is in the form of a capsule, trilayer tablet, mini tablet, gelatin capsule, oral granule or a pellet, preferably a capsule.

5. The pharmaceutical composition according to claim 4, in the form of a capsule comprising dexketoprofen mini tablets and eletriptan mini tablets.

6. The pharmaceutical composition according to claim 5, wherein said dexketoprofen mini tablets comprising 10.00 to 80.00 % by weight dexketoprofen or a pharmaceutically acceptable salt thereof as active agent, and eletriptan mini tablets comprising 10.00 to 90.00 % by weight eletriptan or a pharmaceutically acceptable salt thereof as active agent.

7. The pharmaceutical composition according to claim 5, wherein said dexketoprofen mini tablets or eletriptan mini tablets or both of them comprising at least one coating layer.

8. The pharmaceutical composition according to claim 1, wherein the pharmaceutically acceptable excipient is selected from a group comprising disintegrants, lubricants, fillers, or mixtures thereof.

9. The pharmaceutical composition according to claim 8, wherein said lubricant is selected from a group comprising magnesium stearate, glyceryl behenate, colloidal silicon dioxide, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof, preferably the lubricants are magnesium stearate, sodium stearyl fumarate or mixture thereof.

10. The pharmaceutical composition according to claim 9, wherein the lubricant is sodium stearyl fumarate in dexketoprofen mini tablet and magnesium stearate in eletriptan mini tablet.

11. The pharmaceutical composition according to claim 10, wherein the weight ratio of sodium stearyl fumarate in dexketoprofen mini tablet to magnesium starate in eletriptan mini tablet is between 0.1 and 10, preferably between 0.2 and 5.0, more preferably between 0.5 and 2.0.

12. The pharmaceutical composition according to claim 8, wherein the disintegrant is selected from a group comprising starch, crospovidone (cross-linked polyvinil pyrrolidone), povidone, poloxomer, cross-linked carboxymethyl cellulose (croscarmellose sodium), low-substituted hydroxypropyl cellulose, pregelatinized starch, sodium carboxymethyl cellulose, calcium carboxymethyl cellulose, carboxymethyl cellulose, docusate sodium, guar gum, low substituted hydroxypropyl cellulose, polyacryline potassium, sodium alginate, sodium starch glycolate, alginic acid, alginates, ion-exchange resins, magnesium aluminium silica, sodium dodesyl sulphate, poloxamer, sodium glycine carbonate, sodium lauryl sulphate or mixtures thereof, preferably the disintegrants are cross-linked carboxymethyl cellulose, corn starch, sodium starch glycolate or mixture thereof.

13. The pharmaceutical composition according to claim 10, wherein the disintegrants are corn starch and microcrystalline cellulose in dexketoprofen mini tablet and croscarmellose sodium in eletriptan mini tablet.

14. The pharmaceutical composition according to claim 12 or 13, wherein the weight ratio of total disintegrants in dexketoprofen mini tablet to total disintegrants in eletriptan mini tablet is between 1 and 10.

15. The pharmaceutical composition according to claim 8, wherein the filler is selected from a group comprising microcrystalline cellulose, mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate, polyols, dextrose, maltitol or mixtures thereof, preferably the fillers are microcrystalline cellulose, lactose monohydrate, corn starch or mixture thereof.

16. The pharmaceutical composition according to claim 15, wherein the fillers are microcrystalline cellulose in dexketoprofen mini tablet and microcrystalline cellulose and lactose monohydrate in eletriptan mini tablet.

17. The pharmaceutical composition according to claim 15 or 16, wherein the weight ratio of total fillers in dexketoprofen mini tablet to total fillers in eletriptan mini tablet is between 0.1 and 5, preferably 0.5 and 4, more preferably 0.5 and 2.

18. The pharmaceutical composition according to any of the preceding claims, wherein the pharmaceutically acceptable salt of eletriptan is preferably eletriptan hydrobromide and wherein the pharmaceutically acceptable salt of dexketoprofen is preferably dexketoprofen trometamol.

19. The pharmaceutical composition according to any of the preceding claims, in the form of a capsule comprising;
a. 10.00 to 80.00 % of dexketoprofen or a pharmaceutically acceptable salt thereof
b. 10.00 to 90.00 % of microcrystalline cellulose
c. 3.00 to 50.00 % of corn starch
d. 0.4 to 20.00 % of sodium starch glycolate
e. 0.1 to 10.00 % of sodium stearyl fumarate
by weight of the total dexketoprofen mini tablet
f. 10.00 to 90.00 % of eletriptan or a pharmaceutically acceptable salt thereof
g. 10.00 to 80.00 % of microcrystalline cellulose
h. 10.00 to 70.00 % of lactose monohydrate
i. 0.1 to 15.00 % of croscarmellose sodium
j. 0.1 to 10.00 % of magnesium stearate
by weight of the total eletriptan mini tablet.

20. The process for preparation of the pharmaceutical composition according to claim 19, wherein the process comprising the following steps:
a. Mixing eletriptan hydrobromide, microcrystalline cellulose, lactose monohydrate, croscarmellose sodium and magnesium stearate
b. Compressing the powder mixture to form eletriptan mini tablets
c. Optionally, coating the mini tablets
d. Mixing dexketoprofen trometamol, microcrystalline cellulose, corn starch, sodium starch glycolate and sodium stearyl fumarate
e. Compressing the powder mixture to form dexketoprofen mini tablets
f. Optionally, coating the mini tablets
g. Filling the eletriptan mini tablets and the dexketoprofen mini tablets into capsules.
